# DEMANDE DE BREVET EUROPEEN

(11) **EP 2 036 886 A1**
(43) Date de publication de la demande: **18.03.2009**
(21) Numéro de dépôt: 08290847.6
(22) Date de dépôt: 10.09.2008
(51) Int. Cl.: C07C 311/20, C07D 209/42, A61K 31/18, A61K 31/404

(54) **Association entre un anti-athérothrombotique et un inhibiteur de l'enzyme de conversion de l'angiotensine**

(30) Priorité: 11.09.2007 FR 0706345
(71) Demandeur: Les Laboratoires Servier, 92415 Courbevoie Cedex (FR)
(72) Inventeur: Verbeuren, Tony, 78540 Vernouillet (FR); Sansilvestri-Morel, Patricia, 92160 Antony (FR); Rupin, Alain, 37510 Savonnières (FR); Vallez, Marie-Odile, 93100 Montreuil (FR); Fratacci, Marie-Dominique, 78390 bois d'Arcy (FR); Lerond, Laurence, 78160 Marly-le-Roi (FR); Lavielle, Gilbert, 78170 La Celle Saint Cloud (FR)

(57) **Abrégé**

Association d'un anti-athérothrombotique et d'un inhibiteur de l'enzyme de conversion de l'angiotensine (IECA), ainsi que les compositions pharmaceutiques qui les contiennent.

SEQUENCE LISTING

<110> Les Laboratoires Servier

<120> Association entre un anti-athérothrombotique et un inhibiteur de
l'enzyme de conversion de l'angiotensine

<130> 18886-PERINDO

<160> 12

<170> PatentIn version 3.5

<210> 1
<211> 32
<212> DNA
<213> artificial sequence

<220>
<223> primer

<220>
<221> primer_bind
<222> (1)..(32)

<400> 1
ggatccggta ccgagatggc gtttctccat gt 32

<210> 2
<211> 32
<212> DNA
<213> artificial sequence

<220>
<223> primer

<220>
<221> primer_bind
<222> (1)..(32)

<400> 2
gagcttaagc ttctgtctca ggtcagtata gg 32

<210> 3
<211> 21

<212> DNA
<213> artificial sequence

<220>
<223> primer


<220>
<221> primer_bind
<222> (1)..(21)

<400> 3
agagcagact ttctatttca c                                    21


<210> 4
<211> 19
<212> DNA
<213> artificial sequence

<220>
<223> primer


<220>
<221> primer_bind
<222> (1)..(19)

<400> 4
ccatcttcac aggcatttc                                    19


<210> 5
<211> 19
<212> DNA
<213> artificial sequence

<220>
<223> primer


<220>
<221> primer_bind
<222> (1)..(19)

<400> 5
tgacaaatac actgggaac                                    19

<210> 6
<211> 18
<212> DNA
<213> artificial sequence

<220>
<223> primer


<220>
<221> primer_bind
<222> (1)..(18)

<400> 6
gccaatcttg taggactg                                    18


<210> 7
<211> 21
<212> DNA
<213> artificial sequence

<220>
<223> primer


<220>
<221> primer_bind
<222> (1)..(21)

<400> 7
aagacctcta tgccaacaca g                                21


<210> 8
<211> 18
<212> DNA
<213> artificial sequence

<220>
<223> primer


<220>
<221> primer_bind
<222> (1)..(18)

<400> 8
agccaccgat ccacacag                                    18

<210> 9
<211> 24
<212> DNA
<213> artificial sequence

<220>
<223> primer


<220>
<221> primer_bind
<222> (1)..(24)

<400> 9
agctactgta atgatcagtc aacg 24


<210> 10
<211> 20
<212> DNA
<213> artificial sequence

<220>
<223> primer


<220>
<221> primer_bind
<222> (1)..(20)

<400> 10
agaggtcctt ttcaccagca 20


<210> 11
<211> 19
<212> DNA
<213> artificial sequence

<220>
<223> primer


<220>
<221> primer_bind
<222> (1)..(19)

<400> 11
gccttccgtg ttcctaccc 19

<210> 12
<211> 18
<212> DNA
<213> artificial sequence

<220>
<223> primer

<220>
<221> primer_bind
<222> (1)..(18)

<400> 12
tgcctgcttc accacctt 18

## Description

La présente invention concerne l'association d'un anti-athérothrombotique et d'un inhibiteur de l'enzyme de conversion de l'angiotensine (IECA), ainsi que les compositions pharmaceutiques qui les contiennent.

Plus spécifiquement, la présente invention a pour objet l'association d'un antagoniste spécifique des récepteurs TP et d'un IECA. De manière surprenante, nous avons montré que cette association permettait d'inhiber l'expression du gène de la E-sélectine, molécule d'adhésion de 115kDa intervenant dans le mécanisme de l'inflammation. En effet, la E-sélectine est surexprimée dans les tissus inflammatoires caractéristiques de diverses pathologies telles que le diabète, les maladies athéro-thrombotiques, l'hypertension, l'obésité, la maladie d'Alzheimer, ...

Plus précisément, la E-sélectine favorise l'adhésion réversible entre leucocytes et cellules endothéliales, qui constitue un préalable indispensable à tout processus inflammatoire (Frenette P.S. and Wagner D.D., Insights into selectin function from knockout mice, 1997, Thromb. and Haem., 78, 60-64). Cette étape, dite de « rolling », nécessite l'induction à la surface des cellules endothéliales de molécules d'adhésion de la famille des sélectines (P-sélectine (ou CD62P) et E-sélectine (ou CD62E ou ELAM pour « endothelial leukocyte adhesion molecule »)) qui vont interagir avec leur ligand leucocytaire (« sialylated carbohydrate ligand », « P-selectin glycoprotein ligand 1 » ou PSGL1). Ainsi, la progression des leucocytes roulant sur la paroi endothéliale des vaisseaux se trouve ralentie. S'ensuit une étape d'adhésion ferme, dite de « sticking », au cours de laquelle les leucocytes se fixent à la surface du vaisseau. Enfin, les leucocytes migrent à travers la paroi vasculaire vers les tissus inflammatoires (diapédèse) via un gradient de facteurs chimiotactiques (TNF-α, IL-1, IL-8).

Il est important de noter que l'expression de la E-sélectine est limitée à l'endothélium et répond à des stimuli inflammatoires comme l'IL-1, le TNF-α ou le lipopolysaccharide bactérien (LPS). Le taux de E-sélectine présent à la surface des cellules est maximal 4 à 6 heures après la stimulation. Ce délai est long car elle est synthétisée *de novo* après la stimulation des cellules. Le taux de E-sélectine retrouve son niveau basal 24 heures après activation *mais, in vivo,* dans certaines situations, la E-sélectine persiste plus longtemps à la surface des cellules.

Des formes circulantes des différentes molécules d'adhésion, dont la E-sélectine, existent. Ces formes solubles sont probablement générées par clivage enzymatique sur un site proche du point d'insertion à la membrane. La quantification de ces molécules solubles est corrélée au taux de molécules d'adhésion présent à la surface des cellules endothéliales (Leeuwenberg JFM, Smeets EF, Neefjes JJ et al, E-selectin and intercellular adhesion molecule-1 are released by human endothelial cells in vitro, 1992, Immunology, 77, 543-549). Ainsi, l'augmentation des taux circulants de molécules d'adhésion solubles, et plus particulièrement la E-sélectine, indique une activation des cellules endothéliales (Smith CW, Potential significance of circulating E-selectin, 1997, Circulation, 95, 1986-1988).

Une augmentation du taux de E-sélectine plasmatique a été mise en évidence dans l'obésité et une corrélation positive est démontrée avec l'index de masse corporelle (Ferri C, Desideri G, Valenti, et al, Early upregulation of endothelial adhesion molecules in obese hypertensive men, 1999, Hypertension, 34,568-573). Une augmentation du stress oxydatif dans le système cardiovasculaire de ces patients et/ou des stimuli métaboliques comme par exemple l'insuline au contact de l'endothélium pourraient expliquer ces observations. En effet, une corrélation entre le taux de la E-sélectine et le risque vasculaire est également présente chez des patients diabétiques atteints de diabète de type I ou II (Bannan S, Mansfield MW, Grant PJ, Soluble vascular cell adhesion molecule-1 and E-selectin levels in relation to vascular risk factor and to E-selectin genotype in the first degree relatives of NIDDM patients and in NIDDM patients, 1998, Diabetologia, 41,460-466). Le taux de E-sélectine est d'ailleurs considéré comme marqueur pour les complications vasculaires liées au diabète. La résistance à l'insuline, l'hyperglycémie et l'hyperinsulinémie augmentent l'expression de la E-sélectine expliquant alors la prédisposition à l'athérosclérose observée chez ces patients.

Une augmentation des taux de E-sélectine circulante est largement montrée chez des patients hyperlipidémiques avec une diminution de ces taux après traitement hypolipidémiant (Hackman A, Abe Y, Insull W et al, Levels of soluble cell adhesion molecules in patients with dyslipemia, 1996, Circulation, 93, 1334-1338). Ceci suggère que les taux de cholestérol influencent le niveau de E-sélectine soluble. En effet, une dyslipidémie sévère entraîne une dysfonction endothéliale avec augmentation de l'expression de E-sélectine dans les cellules endothéliales. De nombreuses études ont montré des corrélations entre le taux et expression de E-sélectine et l'hypercholesterolémie et l'athérosclérose. La synthèse de E-sélectine étant induite par des cytokines, une augmentation du niveau de E-sélectine pourrait être un marqueur d'inflammation vasculaire.

De nombreuses études ont également mis en évidence chez des patients atteints de maladies veineuses chroniques une activation des cellules endothéliales due à l'hypertension veineuse et donc une élévation des taux circulants de molécules d'adhésion (Saharay M, Shields DA, Georgiannos SN et al, Endothelial activation in patients with chronic venous disease, 1998, Eur J Vasc Surg, 15, 342-349 ; Verbeuren TJ, Bouskela E, Cohen RA et al, Regulation of adhesion molecules : a new target for the treatment of chronic venous insufficiency, 2000, Microcirculation, 7,S41-S48).

Par ailleurs, l'augmentation du taux de E-sélectine a aussi été décrite chez des patients hypertendus (Ferri C, Bellini C, Desideri G et al, Clustering of endothelial markers of vascular damage in human salt-sensitive hypertension. Influence of dietary sodium load and depletion, 1998, Hypertension, 32,862-868).

De même, de nombreuses références de la littérature font état du rôle de la E-sélectine dans les complications du système rénal (Singbartl K, Ley K, Protection from ischemia-reperfusion induced severe acute renal failure by blocking E-selectin, 2000, Crit. Care. Med., 28(7), 2507-2514, Nakatani K, Fujii H, Hasegawa H et al, Endothelial adhesion molecules in glomerular lesions: association with their severity and diversity in lupus models, 2004, Kidney Int., 65(4), 1290-1300).

L'implication de la E-sélectine chez des patients atteints de la maladie d'Alzheimer a également été démontrée; en effet, des taux élevés de la E-sélectine ont été observés chez ces patients ( Borroni B, Volpi R, Martini G, Del Bono R, Archetti S, Colciaghi F, Akkawi NM, Di Luca M, Romanelli G, Caimi L, and Padovani A, Peripheral blood abnormalities in Alzheimer Disease : Evidence for early endothelial dysfunction, 2002, Alzheimer's disease and Associated disorders, 16 (3), 150-155).

Enfin, de nombreuses revues établissent l'implication du taux de E-sélectine dans les processus métastatiques, et donc dans le cancer (Kobayashi H, Boelte KC, Lin PC, Endothelial Cell Adhesion Molecules and Cancer Progression, 2007, Current Medical Chemistry, 14,377-386 ; Kneuer C, Ehrhardt C, Radomski MW, Bakowsky U, Selectins - potential pharmacological targets ?, 2006, Drug Discovery Today, Vol.11, N°21/22, 1034-1040).

La présente invention concerne l'association d'un anti-athérothrombotique et d'un inhibiteur de l'enzyme de conversion de l'angiotensine (IECA) dans laquelle:
- le composé anti-athérothrombotique est un composé (A) de formule (I) : 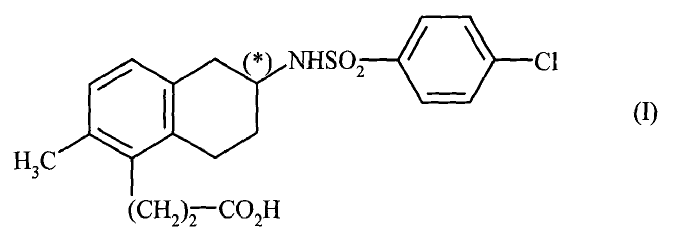 sous forme racémique ou d'isomère optiquement pur ainsi que ses sels d'addition pharmaceutiquement acceptables. Décrit dans le brevet EP 648741, ce composé est un puissant antagoniste des récepteurs TP, plus particulièrement un antagoniste spécifique du thromboxane A₂ et des récepteurs des prostaglandines-endoperoxides (PGG₂-PGH₂). Par ailleurs, il a été montré que ce composé diminuait significativement l'expression endothéliale de la molécule d'adhésion VCAM-1 chez des souris athéromateuses Apo E^{-/-} diabétiques (Zuccollo A, Shi C, Mastroianni R et al, The thromboxane A2 receptor antagonist S 18886 prevents enhanced atherogenesis caused by diabetes mellitus, 2005, Circulation, 112, 3001-3008).
- l'inhibiteur de l'enzyme de conversion de l'angiotensine (IECA) est choisi non limitativement parmi les composés suivants : le perindopril éventuellement sous forme de son métabolite actif le perindoprilate, le ramipril éventuellement sous forme de son métabolite actif le ramiprilate, l'enalapril éventuellement sous forme de son métabolite actif le l'enalaprilate, le captopril, le lisinopril, le delapril, le fosinopril, le quinapril, le spirapril, l'imidapril, le trandolapril éventuellement sous forme de son métabolite actif le trandolaprilate, le benazepril, le cilazapril, le temocapril, l'alacepril, le ceronapril, le moveltipril ou le moexipril, ainsi que leurs sels d'addition à un acide ou à une base pharmaceutiquement acceptable. On peut noter que certains IECA inhibent l'induction de l'expression des molécules d'adhésion dans les cellules endothéliales des souris Apo E^{-/-} infusées avec de l'angiotensine II (da Cunha V, Tham DM, Martin-McNulty B et al, Enalapril attenuates angiotensin II-induced atherosclerosis and vascular inflammation, 2005, Atherosclerosis, 178, 9-17).

En étudiant l'interaction entre les systèmes thromboxane A₂-TP récepteur et rénine-angiotensine, nous avons démontré une synergie importante de ces voies sur l'expression des molécules d'adhésion.

Pour cela, un antagoniste des récepteurs TP a été utilisé à une concentration sans effet sur l'expression de la E-sélectine induite par du TNF-α dans des cellules endothéliales humaines. Cette même concentration a été ensuite testée en présence de différentes concentrations également inactives de plusieurs IECA. Une diminution significative de l'expression de la E-sélectine induite par du TNF-α dans des cellules endothéliales humaines a alors été observée avec l'association des deux composés, démontrant une synergie non prévisible entre les deux composés.

Cet effet synergique a également été mis en évidence dans un test de thrombose et de pression artérielle chez le rat. Lors de ce test, il a été montré que l'activité antithrombotique du composé (A) de formule (I) est potentialisée en présence du composé (B) et augmente de manière extrêmement importante et totalement non prévisible. Ce test démontre également que la présence du composé (A) de formule (I) potentialise de façon importante et non prévisible l'effet anti-hypertenseur du composé (B).

L'association du composé (A) de formule (I) et le composé (B) a également permis de diminuer nettement et significativement l'expression de vascular cell adhesion molecule 1 (VCAM-1) dans l'aorte et de la fibronectine dans le rein dans un modèle de souris athéromateuses diabétiques.

Ces résultats permettent d'envisager l'utilisation d'une association [ antagoniste des récepteurs TP / IECA ] pour la fabrication d'un médicament utile dans le traitement des complications vasculaires, et plus particulièrement cardiovasculaires et cérébrovasculaires, liées au diabète, aux maladies athéro-thrombotiques, à l'hyperlipidémie, à l'hypertension, aux maladies veineuses chroniques, à l'inflammation, au syndrome métabolique lié à l'obésité, ou au cancer. Parmi les maladies athéro-thrombotiques, les compositions selon l'invention sont particulièrement utiles pour le traitement de l'infarctus du myocarde, de l'angine de poitrine, des accidents vasculaires cérébraux, les anévrismes aortiques ou de l'artérite des membres inférieurs. Par ailleurs, la néphropathie liée au diabète, à l'hypertension ou aux maladies inflammatoires est également une indication dans laquelle l'association [ antagoniste des récepteurs TP / IECA ] est particulièrement utile. Enfin, la rétinopathie diabétique fait aussi partie des indications thérapeutiques préférées de cette invention.

Les facteurs de risques vasculaires et les maladies vasculaires, tels que l'hypertension, l'obésité, le diabète, les maladies cardiaques, les maladies cérébrovasculaires et l'hyperlipidémie et donc l'athérosclérose sont impliqués dans la genèse des démences telles que la maladie d'Alzheimer et la démence vasculaire (Qiu C., De Ronchi D. et Fratiglioni L., The epidemiology of the dementias : an update, 2007, Current Opinion in Psychiatry, 20, 380-385,). De plus, dans les maladies neurodégénératives comme la maladie d'Alzheimer, une augmentation des taux d'isoprostanes a été observée. Ces isoprostanes sont des marqueurs, mais également des médiateurs du stress oxidant qui serait à l'origine de la maladie (Montuschi P., Barnes PJ. et Jackson Roberts II L., Isoprostanes: markers and mediators of oxidative stress, 2004, The FASEB Journal, 18, 1791-1800). Les isoprostanes exercent au moins une partie de leur activité en stimulant les récepteurs TP (Montuschi P., Bames PJ. et Jackson Roberts II L., Isoprostanes: markers and mediators of oxidative stress, 2004, The FASEB Journal, 18, 1791-1800). et leur activité serait donc bloquée par la présente association.
Notre association agît, comme démontré par les études décrites dans la présente demande de brevet, sur les maladies vasculaires qui sont des facteurs de risque pour les démences.

Les IECA préférés sont le perindopril de formule (B) et le ramipril de formule (C) ainsi que leurs sels, et plus particulièrement le perindopril de formule (B) et ses sels :

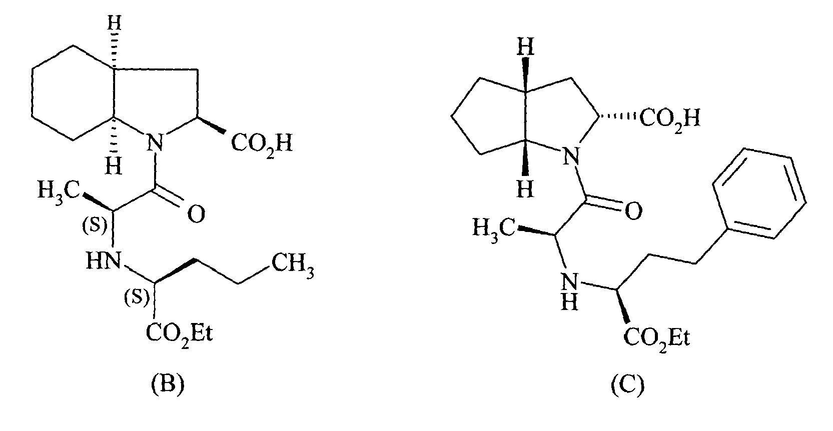

Parmi les sels d'addition du perindopril, on peut citer à titre non limitatif les sels d'addition à une base pharmaceutiquement acceptable comme les sels de *tert*butylamine, d'arginine, de sodium, de potassium, etc...

Le perindopril se présentera préférentiellement sous forme d'un sel de *tert*butylamine ou d'un sel d'arginine.

Dans les associations selon l'invention, le composé (A) est préférentiellement l'acide 3-[(6R)-6-[[(4-chlorophényl)sulfonyl] amino]-2-méthyl-5,6,7,8-tétrahydronaphtalèn-1-yl] propanoïque, aussi appelé terutroban. Des associations analogues impliquant d'autres antagonistes des TP récepteurs comme l'ifetroban ou le ramatroban sont aussi envisageables.

Parmi les sels d'addition du composé (A), on peut citer à titre non limitatif les sels d'addition à une base pharmaceutiquement acceptable comme les sels de sodium, de potassium, de *tert*butylamine, de diéthylamine, etc... Le sel de sodium du terutroban sera plus particulièrement préféré.

Dans les compositions pharmaceutiques selon l'invention, les quantités d'IECA et d'antagoniste des récepteurs TP sont adaptées à la nature de ces principes actifs et leurs proportions relatives sont donc variables en fonction des principes actifs.

Lorsque le composé (A) est le terutroban sous forme de sel de sodium et que l'IECA est le perindopril sous forme de sel de *tert*butylamine ou d'arginine, ces proportions sont comprises entre 10 et 40% de la masse totale des principes actifs pour le perindopril et entre 60 et 90% de la masse totale des principes actifs pour le terutroban.

Les pourcentages préférés pour cette association sont compris entre 15 et 25% de perindopril sous forme de sel de t*ert*butylamine contre 75 à 85% de terutroban sous forme de sel de sodium, et compris entre 20 et 30% de perindopril sous forme de sel d'arginine contre 70 à 80% de terutroban sous forme de sel de sodium.

La présente invention concerne également les compositions pharmaceutiques renfermant une association du composé (A) et d'un IECA, un ou les deux éventuellement sous forme de sels pharmaceutiquement acceptables avec un ou plusieurs véhicules ou excipients inertes, non toxiques et appropriés.

Dans les compositions pharmaceutiques selon l'invention, la fraction massique en principes actifs (masse des principes actifs sur la masse totale de la composition) est comprise entre 5 et 50%.

En ce qui concerne les excipients pharmaceutiquement acceptables, on peut citer à titre non limitatif les liants, les diluants, les délitants, les stabilisants, les conservateurs, les lubrifiants, les odorants, les aromatisants ou les édulcorants.

Parmi les compositions pharmaceutiques selon l'invention, on retiendra plus particulièrement celles qui conviennent pour l'administration par voie orale, parentérale et notamment intraveineuse, per ou transcutanée, nasale, rectale, perlinguale, oculaire, respiratoire et plus spécifiquement les comprimés simples ou dragéifiés, les comprimés sublinguaux, les gélules, les glossettes, les capsules, les tablettes, les préparations injectables, les aérosols, les gouttes oculaires ou nasales, les suppositoires, les crèmes, pommades, gels dermiques, etc...

La voie d'administration préférée est la voie orale et les compositions pharmaceutiques correspondantes peuvent permettre la libération instantanée ou différée des principes actifs.

Les compositions pharmaceutiques préférées sont les comprimés.

La posologie est adaptable selon la nature et la sévérité de l'affection, la voie d'administration ainsi que l'âge et le poids du patient. Dans les compositions selon l'invention, elle s'échelonne entre 1 et 100 mg pour le composé (A) et entre 0.5 et 100 mg selon la nature de l'IECA en une ou plusieurs prises par 24 heures. Lorsque l'IECA est le perindopril, la dose d'administration journalière est comprise entre 0.5 et 20 mg en une ou plusieurs prises.

Les exemples de composition ci-dessous sont donnés à titre non limitatif.

### Comprimés de terutroban / perindopril :

### EXEMPLE 1 :

| *Constituants* | *Quantité (mg)* |
|---|---|
| Terutroban, sel de sodium | 30 |
| perindopril, sel de tertbutylamine | 8 |
| silice colloïdale hydrophobe | 0.4 |
| amidon | 6 |
| stéarate de magnésium | 2 |
| cellulose microcristalline | 50 |
| lactose | 103.6 |
| Pour un comprimé terminé à | 200 |

### EXEMPLE 2 :

| *Constituants* | *Quantité (mg)* |
|---|---|
| Terutroban, sel de sodium | 30 |
| perindopril, sel d'arginine | 10 |
| silice colloïdale hydrophobe | 0.4 |
| amidon | 6 |
| stéarate de magnésium | 2 |
| cellulose microcristalline | 50 |
| lactose | 101,6 |
| Pour un comprimé terminé à | 200 |

### RESULTATS PHARMACOLOGIQUES

### Inhibition in vitro de l'expression de la E-sélectine

### 1) Culture cellulaire

L'étude est réalisée sur des cellules endothéliales humaines HUVEC (*Human Umbilical Vein Endothelial Cells,* Clonetics Co). Les cellules sont cultivées dans un milieu EBM2 *(Endothelial Basal Medium,* Clonetics Co) supplémenté avec 2% SVF (*Sérum de Veau Foetal*) et EGM2 (*Endothelial Growth Medium,* Clonetics Co).

### 2) Clonage du promoteur E-sélectine

### a) amplification du promoteur par PCR

Un fragment de 850pb, correspondant au promoteur humain E-sélectine, s'étendant des nucléotides en position -800 à +50 (n° accession M64485 ; Tamaru et al., E-selectin gene expression is induced synergistically with the coexistence of activated classic protein kinase C and signals elicited by interleukin-1β but not tumor necrosis factor-α, 1999, J. Biol. Chem, 274, 3753-3763), a été amplifié par PCR et sous-cloné. Dans un volume réactionnel final de 100 µl, 5 unités de *nativePyrococcus furiosus (Pfu)* DNA polymerase (Stratagene) sont mis en présence de 1 µg d'ADN génomique humain (Clontech), 200µM de dNTP (deoxynucléotides triphosphate) (Clontech), 200ng d'amorces dans un milieu contenant le tampon spécifique de l'enzyme. Le jeu d'amorces utilisé est le suivant :
5'-GGATCCGGTACCGAGATGGCGTTTCTCCATGT (SEQ ID N°1) et
5'-GAGCTTAAGCTTCTGTCTCAGGTCAGTATAGG (SEQ ID N°2)
Le programme de PCR, sur appareil Gene Amp PCR system 9700, présente une initiation à 94°C pendant 1 min puis une amplification sur 35 cycles (94°C pendant 1 min, 55°C pendant 1 min, 72°C pendant 3 min). Le produit de PCR est ensuite précipité toute la nuit à -20°C en présence d'acétate de sodium 0.3 M pH 5,2 et d'éthanol. Après centrifugation à 14000 rpm à 4°C, le culot est resuspendu dans l'éthanol 70% et de nouveau centrifugé à 14000 rpm à 4°C. Le culot obtenu est alors séché puis repris dans l'eau.

### b) digestion de la séquence promotrice

La séquence amplifiée est ensuite digérée en deux étapes par les enzymes de restriction Kpn I et Hind III. La séquence amplifiée est digérée 1h30 à 37°C en présence de 30 unités d'enzyme et 100 µg/ml de BSA (*Bovine Serum Albumin*). Après chaque digestion, le produit obtenu est systématiquement purifié sur colonnes Micro Bio-Spin^{®} Chromatography (Bio-Rad) afin d'éliminer les sels du tampon.

### c) construction du plasmide PGL3 / E-sélectine

Le plasmide pGL3 Basic (Promega), contenant le gène luciférase de la luciole Firefly, est digéré par les enzymes de restriction Kpn I et Hind III selon le même protocole que pour l'insert puis purifié sur gel d'agarose Low Melting 1 %.
La ligation du vecteur plasmidique pGL3-Basic et de l'insert correspondant au promoteur de la E-sélectine a été réalisée par la T4 DNA Ligase (LigaFast™ Rapid DNA Ligation System de Promega). Par convention, lors d'une ligation, il est utilisé un excédent d'insert équivalent au triple de la quantité de vecteur. De plus, cet insert étant six fois plus court que le vecteur (0,85kb contre 4,8kb), le maintien de l'équilibre stoechiométrique nécessite une masse six fois supérieure de vecteur. Il a donc été mis en réaction, 10,6 ng d'insert et 25 ng de vecteur pGL3 Basic en présence de 3 unités de T4 ligase, à température ambiante.

### 3) Transfection de cellules HUVEC

Le plasmide PGL3/E-sélectine est transfecté dans des cellules HUVEC avant le quatrième passage. La transfection a lieu dans des plaques présentant des cellules à 50% de confluence, en déposant dans chacun des puits de la Lipofectin® (Invitrogen) et 3 µg de plasmide PGL3/E-sélectine. La lipofectin® (6µg/ml) est préalablement activée durant 30 min dans un milieu OPTI-MEM (GIBCO^{™}) puis mis en contact 15 min avec les plasmides préalablement dilués dans le milieu. Les cellules sont incubées 4 heures à 37°C dans une atmosphère à 5% CO₂ et 95% O₂. Le milieu de transfection est retiré et remplacé pendant la nuit par un milieu de culture enrichi afin de stabiliser les cellules.

L'induction de l'expression des gènes rapporteurs est réalisée durant 4h dans un milieu sans sérum M 199 (GIBCO^{™}). Les cellules sont grattées et lysées dans un tampon de lyse (kit Dual-Luciferase®, Promega) puis conservées à -20°C.

La phase d'induction est de 4h pour les cellules HUVEC, en présence de 100U/ml Tumor Necrosis Factor-α (TNF-α). Durant cette phase inductive, il est ajouté différentes concentrations d'une part :
- de périndoprilate (0 à 100µM), de sel de sodium du terutroban (0 à 100µM) ou sel de sodium du terutroban (30µM) + différentes concentrations de périndoprilate (10, 30 et 100µM) (tableau 1)
   et d'autre part :
- de ramiprilate (0 à 100µM), de sel de sodium du terutroban (0 à 100µM) ou de sel de sodium du terutroban (10µM) + différentes concentrations de ramiprilate (30 et 100µM) (tableau 2).

### 4) Détermination de l'activité du promoteur

L'activité du promoteur E-sélectine est déterminée par une quantification de l'activité luciférase produite (kit Dual-Luciferase®, Promega). Dans chaque puits est ajouté une solution de luciférine, le substrat de la luciférase Firefly. Il en résulte une émission de lumière. La plaque est incubée 10 min à l'obscurité puisque l'activité luciférase est sensible à la lumière, puis débute une lecture au luminomètre pour quantifier les photons émis (Wallac, Perkin Elmer), le résultat obtenu étant la moyenne de cpm (coups par minute) sur une période de 5s.

### 5) Résultats terutroban - perindopril

Le terutroban (composé A) sous forme de sel de sodium et le perindopril (composé B) sous forme de son métabolite actif le perindoprilate, ont été testés séparément à différentes concentrations (0, 10, 30, 100µM) sur les cellules HUVEC après induction avec le TNF-α. De manière analogue, le composé A sous forme de sel de sodium (30µM) + différentes concentrations de composé B sous forme de son métabolite actif (0, 10, 30, 100µM) ont été étudiées. L'activité du promoteur E-sélectine est mesurée en condition contrôle et en présence de produits en état induit. Les activités, exprimées en cpm et en pourcentage des observations contrôles, sont illustrées dans le tableau 1.

**Tableau 1: Mesure de l'activité du promoteur E-sélectine en présence de sel de sodium de composé A, du métabolite actif de composé B ou de sel de sodium de composé A (30µM) + métabolite actif de composé B en condition induite par TNF-α (100U/ml). *p<0.05 ; ** p<0.01 par rapport au contrôle ANOVA 1 facteur, post test Dunnett (n=4-5).**

| | | **Activité en cpm % / Contrôle MOY ± ESM** | **% inhibition/contrôle** |
|---|---|---|---|
| **Composé A, sel de sodium** | Contrôle | 100% | |
| | 10µM | 105.8 ± 7.9 | |
| | 30µM | 99.5 ± 9.1 | |
| | 100µM | 88.8 ± 8.9 | |
| **Composé B, métabolite actif** | Contrôle | 100% | |
| | 10µM | 99.5 ± 7.8 | |
| | 30µM | 100.3 ± 9.2 | |
| | 100µM | 82.8 ± 11.5 | |
| **Composé A, sel de sodium (30µM) Composé B, métabolite actif** | Contrôle | 100% | |
| | 10µM | 60.8 ± 8.1 (**) | 41.7 ± 10.1 |
| | 30µM | 67.1 ± 6.0 (*) | 35.4 ± 5.0 |
| | 100µM | 47.6 ± 8.7 (**) | 52.5 ± 8.5 |

Le sel de sodium du terutroban et le perindopril sous forme de son métabolite actif le périndoprilate n'ont aucun effet sur l'expression du gène E-sélectine aux concentrations testées. Lorsque le périndoprilate est co-incubé avec le sel de sodium du terutroban (30µM), une inhibition de l'expression du gène E-sélectine est alors observée dès 10µM de périndoprilate (60.8% d'activité d'expression du gène E-sélectine versus 100% sans produit; p<0.01, ANOVA 1 facteur, post test Dunnett).

Les résultats montrent très clairement que l'administration de ces deux composés en association permet d'obtenir un effet synergique important tout à fait inattendu.

### 6) Résultats terutroban - ramipril

Le terutroban (composé A) sous forme de sel de sodium et le ramipril (composé C) sous forme de son métabolite actif le ramiprilate, ont été testés séparément à différentes concentrations (0, 30, 100µM) sur les cellules HUVEC après induction avec le TNF-α. De manière analogue, le composé A sous forme de sel de sodium (10µM) + différentes concentrations de composé C sous forme de son métabolite actif (0, 30, 100µM) ont été étudiées. L'activité du promoteur E-sélectine est mesurée en condition contrôle et en présence de produits en état induit. Les activités, exprimées en cpm et en pourcentage des observations contrôles, sont illustrées dans le tableau 2.

**Tableau 2: Mesure de l'activité du promoteur E-sélectine en présence de sel de sodium de composé A, du métabolite actif de composé C ou de sel de sodium de composé A (10µM) + métabolite actif de composé C en condition induite par TNF-α (100U/ml).**

| | | **Activité en cpm % / Contrôle MOY ± ESM** | **% inhibition/contrôle** |
|---|---|---|---|
| **Composé A, sel de sodium** | Contrôle | 100% | |
| | 30µM | 99.5 ± 9.1 | |
| | 100µM | 88.8 ± 8.9 | |
| **Composé C, métabolite actif** | Contrôle | 100% | |
| | 30µM | 111.1 ± 17.2 | |
| | 100µM | 114.6 ± 38.9 | |
| **Composé A, sel de sodium (10µM)** + **Composé C, métabolite actif** | Contrôle | 100% | |
| | 30µM | 55.2 ± 4.8 (**) | 44.7 ± 4.8 |
| | 100µM | 44.2 ± 9.1 (**) | 55.8 ± 9.1 |

| | | | |
|---|---|---|---|
| ** p<0.01 par rapport au contrôle ANOVA 1 facteur, post test Dunnett (n=3). | | | |

Le terutroban sous forme de sel de sodium et le ramipril sous forme de son métabolite actif le ramiprilate n'ont aucun effet sur l'expression du gène E-sélectine aux concentrations testées. Lorsque le ramiprilate est co-incubé avec le sel de sodium du terutroban (10µM), une inhibition de l'expression du gène E-sélectine est alors observée dès 30µM de ramiprilate (55.2% d'activité d'expression du gène E-sélectine versus 100% sans produit; p<0.01, ANOVA 1 facteur, post test Dunnett).

Les résultats montrent très clairement que l'administration de ces deux composés en association permet d'obtenir un effet synergique important tout à fait inattendu.

Les résultats de l'association d'une part du terutroban (composé A) sous forme de sel de sodium et perindopril (composé B) sous forme de son métabolite actif et d'autre part du terutroban (composé A) sous forme de sel de sodium et du ramipril (composé C) sous forme de son métabolite actif montrent que l'association entre le composé (A) de formule (I) et d'un inhibiteur de l'enzyme de conversion de l'angiotensine présente un effet synergique important tout à fait inattendu.

### Inhibition de la thrombose et de la pression artérielle chez le rat

### 1) Matériel et méthodes

La technique de thrombose utilisée est celle de Tanaka et collaborateurs (Eur. J. Pharmacol., 2008 ; 401, : 413-18).

### a) Thrombose artérielle

Des rats CD (350-375 g) sont anesthésiés avec le pentobarbital 50 mg/kg IP et placés sur une couverture thermorégulée et thermostatée. Après laparotomie l'aorte est dégagée. La thrombose est induite en déposant une pastille de papier filtre (8mm) saturé en FeCl₃ à 50% pendant 10 minutes. 20 minutes après le retrait de la pastille l'artère est ligaturée et incisée, le caillot existant est pesé. Certains animaux sont traités avec le terutroban (composé A) sous forme de sel de sodium à la dose de 0.1 mg/kg, d'autres avec le périndopril (composé B) sous forme de sel de *tert*butylamine à la dose de 1 mg/kg et d'autres avec le terutroban sous forme de sel de sodium (composé A) à 0.1 mg/kg et le périndopril (composé B) sous forme de sel de *tert*butylamine à 1 mg/kg.

### b) Pression artérielle

Après anesthésie au pentobarbital (50mg/kg IP), l'animal est placé sur une couverture thermorégulée et thermostatée. La carotide est dégagée et un cathéter introduit afin de suivre la pression artérielle à l'aide d'un capteur Gould relié à un logiciel Acqknowledge. La pression artérielle est enregistrée 1 heure après le traitement et pendant 30 minutes. Certains animaux sont traités avec le terutroban (composé A) sous forme de sel de sodium à la dose de 0.1mg/k d'autres avec le périndopril (composé B) sous forme de sel de *tert*butylamine à la dose de 0.3 mg/kg et d'autres avec le terutroban (composé A) sous forme de sel de sodium à 0.1mg/kg et le périndopril (composé B) sous forme de sel de *tert*butylamine à 0.3 mg/kg.

### 2) Résultats

### a) Thrombose artérielle

Les poids de caillots chez les rats contrôles sont de 17.1 ± 1.3 mg ; le traitement avec le terutroban (composé A), sel de sodium à 0.1 mg/kg n'a pas modifié ce poids : 16.8 ±1.3 mg. Les poids de caillots chez les rats contrôles sont de 15.6 ± 0.7 mg ; le traitement avec le perindopril (composé B), sel de *tert*butylamine à 1 mg/kg n'a pas modifié ce poids : 15.2 ±1.1 mg. Les poids de caillots chez les rats contrôles sont de 16.3 ± 0.8 mg ; le traitement avec l'association du terutroban (composé A), sel de sodium à 0.1mg/kg avec le périndopril (composé B), sel de *tert*butylamine à mg/kg a significativement diminué le poids du caillot jusqu'à 10.1 ± 0.6 mg.
Ces résultats mettent en évidence la synergie d'action inattendue des deux substances au niveau de la thrombose artérielle.

### b) Pressions artérielles

La pression artérielle des rats contrôles est de 131 ± 7 mmHg. Ni le terutroban (composé A), sel de sodium à 0.1 mg/kg, ni le périndopril (composé B), sel de *tert*butylamine à 0.3 mg/kg (qui est une dose inactive chez le rat) n'ont modifié cette pression : 126 ± 7 mmHg et 120 ± 9 mmHg respectivement. L'association du terutroban (composé A), sel de sodium et du périndopril (composé B), sel de *tert*butylamine a fortement et significativement baissé la pression artérielle jusqu'à 95 ± 7 mmHg.
Ces résultats démontrent la synergie d'action inattendue des deux substances au niveau de la régulation de la pression artérielle.

Ce test démontre les activités inhibitrices au niveau de la thrombose et de la pression artérielle de l'association du terutroban (composé A) et du périndopril (composé B) et illustre donc le potentiel pour le traitement, avec cette association, des pathologies artérielles telles que les maladies thrombotiques (infarctus du myocarde, angine de poitrine, accidents vasculaires cérébraux, artérites des membres inférieurs,...) et l'hypertension.

### Inhibition de l'expression de Vascular Cell Adhesion Molecule 1 (VCAM-1) aortique et de la fibronectine rénale in vivo

Quatre groupes de 9 souris déficientes en apolipoprotéine E (ApoE^{-/-}, développant spontanément des plaques d'athérome dans les aortes) sont utilisés dans cette étude. A l'âge de 8 semaines, les souris sont rendues diabétiques par 5 injections intra-péritonéales de 70 mg/kg de streptozotocine sur 5 jours. A la neuvième semaine, les animaux sont partagés en quatre groupes : un groupe contrôle non traité, un groupe traité avec le terutroban (composé A), sel de sodium (1 mg/kg/jour dans la nourriture), un groupe traité avec le périndopril (composé B), sel de *tert*butylamine (0.1 mg/kg/jour dans l'eau de boisson), un groupe traité avec l'association du terutroban (composé A), sel de sodium (1 mg/kg/jour dans la nourriture) et le périndopril (composé B), sel de tertbutylamine (0.1 mg/kg/jour dans l'eau de boisson).

Pour l'expression de la fibronectine rénale, les souris sont traitées 6 semaines puis sacrifiées après anesthésie à l'isoflurane.

Pour l'expression de VCAM-1 aortiques, les souris sont traitées 13 semaines puis sacrifiées. Les aortes et les reins droits sont prélevés, disséqués et congelés dans l'azote liquide.

Les tissus sont cryobroyés, et les ARN totaux sont extraits à l'aide du kit RNeasy® micro (Qiagen). La transcription inverse est ensuite réalisée à partir de 1µg d'ARN total à l'aide du kit Superscript^{™} III first-strand cDNA synthesis (Invitrogen). Les expressions de VCAM-1 aortique et de la fibronectine rénale sont quantifiées par PCR en temps réel et normalisées par rapport à 3 gènes de référence : β-actine, hypoxanthine-guanine phosphoribosyl transferase (HPRT) et glyceraldehyde phosphate deshydrogenase (GAPDH). Le kit IQ^{™} SYBR® Green supermix (Biorad) est utilisé, avec 2µl d'ADNc et 150 nM de chaque amorce. Les échantillons sont dénaturés 5 minutes à 95°C et amplifiés durant 40 cycles selon le protocole suivant : dénaturation pendant 20 secondes à 95°C et hybridation et élongation pendant 1 minute à 52° C pour fibronectine, 54°C pour VCAM-1, β-actine et HPRT, 56°C pour GAPDH. Le cycle seuil (défini comme celui pour lequel la fluorescence est considérée comme significativement plus élevée que le bruit de fond) pour VCAM-1 aortique et fibronectine rénale des animaux non traités est normalisé par rapport aux gènes de référence (et considéré comme 100%) puis comparé à celui des animaux traités.
Les amorces spécifiques utilisées sont les suivantes :
VCAM-1 :
   5'-AGA GCA GAC TTT CTA TTT CAC-3' (sens) (SEQ ID N°3) et
   5'-CCA TCT TCA CAG GCA TTT C-3' (antisens) (SEQ ID N°4);
Fibronectine :
   5'-TGA CAA ATA CAC TGG GAA C-3'(sens) (SEQ ID N°5) et
   5'-GCC AAT CTT GTA GGA CTG-3' (antisens) (SEQ ID N°6);
β actine :
   5'-AAG ACC TCT ATG CCA ACA CAG-3' (sens) (SEQ ID N°7) et
   5'-AGC CAC CGA TCC ACA CAG-3' (antisens) (SEQ ID N°8);
HPRT :
   5'-AGC TAC TGT AAT GAT CAG TCA ACG-3' (sens) (SEQ ID N°9) et
   5'-AGA GGT CCT TTT CAC CAG CA-3' (antisens) (SEQ ID N° 10);
GAPDH :
   5'-GCC TTC CGT GTT CCT ACC C-3' (sens) (SEQ ID N°11) et
   5'-TGC CTG CTT CAC CAC CTT-3' (antisens) (SEQ ID N°12).

L'activité des composés du terutroban (composé A), sel de sodium, du périndopril (composé B), sel de *tert*butylamine et de leur association est évaluée en comparant les niveaux d'expression de VCAM-1 aortique et fibronectine rénale à ceux des animaux non traités (considérés comme 100%).

### Fibronectine rénale :

Le traitement des souris avec le terutroban (composé A), sel de sodium ou le périndopril (composé B), sel de *tert*butylamine seuls n'a pas d'effet significatif sur l'expression du gène de la fibronectine rénale (69 ± 16% pour le terutroban (composé A), sel de sodium et 86 ± 9.9% pour le périndopril (composé B), sel de *tert*butylamine *versus* 100% sans traitement, NS, ANOVA 1 facteur, post test Dunnett). Lorsque les souris sont traitées avec l'association du terutroban (composé A), sel de sodium et du périndopril (composé B), sel de *tert*butylamine, une inhibition de l'expression du gène de la fibronectine est alors observée (43 ± 9.1 % *versus* 100% sans traitement, P<0.01, ANOVA 1 facteur, post test Dunnett).

### VCAM-1 aortique:

Les souris traitées avec le terutroban (composé A), sel de sodium ou le périndopril (composé B), sel de *tert*butylamine seuls n'ont pas d'effet significatif sur l'expression du gène VCAM-1 aortique (l'expression restante est de 88 ± 22% pour le terutroban (composé A), sel de sodium et 76 ± 21% pour le périndopril (composé B), sel de *tert*butylamine *versus* 100% sans traitement, NS, ANOVA 1 facteur, post test Dunnett). Lorsque les souris sont traitées avec l'association du terutroban (composé A), sel de sodium et du périndopril (composé B), sel de tertbutylamine, une inhibition de l'expression du gène VCAM-1 est alors observée (l'espression restante est de 27 ± 6.2 % *versus* 100% sans traitement, P<0.01, ANOVA 1 facteur, post test Dunnett).

Le traitement des animaux athéromateux et diabétiques par l'association terutroban (composé A), sel de sodium/périndopril (composé B), sel de *tert*butylamine permet donc de diminuer nettement et significativement l'expression de VCAM-1 dans l'aorte et de la fibronectine dans le rein et cela par rapport aux animaux non traités, ou traités avec le terutroban (composé A), sel de sodium ou le périndopril (composé B), sel de *tert*butylamine seuls. Les résultats montrent très clairement que l'administration de ces deux composés en association permet d'obtenir un effet synergique important tout à fait inattendu.

Ce test démontre les activités inhibitrices sur l'expression des molécules d'adhésion et sur la fibronectine rénale de l'association terutroban (composé A)/périndopril (composé B) et donc un potentiel pour le traitement des pathologies artérielles telles que les complications vasculaires liées au diabète, l'hypertension, l'athérosclérose, l'inflammation, le syndrome métabolique lié à l'obésité, les complications vasculaires liées à l'obésité, l'angine de poitrine, artérites des membres inférieurs, accidents vasculaires cérébraux. Vu le rôle que jouent les molécules d'adhésion dans la maladie veineuse, l'association peut également traiter cette maladie.

## Revendications

1. Association du composé (A) de formule (I) éventuellement sous forme d'isomère optique ou d'un de ses sels pharmaceutiquement acceptable et d'un inhibiteur de l'enzyme de conversion de l'angiotensine ou d'un de ses sels pharmaceutiquement acceptable :

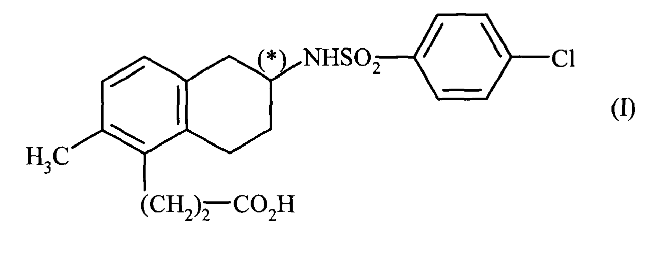

2. Association selon la revendication 1 **caractérisée en ce que** le composé (A) est le terutroban.

3. Association selon l'une quelconque des revendications 1 ou 2 **caractérisée en ce que** le composé (A) est sous la forme d'un sel de sodium.

4. Association selon l'une quelconque des revendications 1, 2 ou 3 **caractérisée en ce que** l'inhibiteur de l'enzyme de conversion de l'angiotensine est le perindopril éventuellement sous forme de son métabolite actif le perindoprilate, le ramipril éventuellement sous forme de son métabolite actif le ramiprilate, l'enalapril éventuellement sous forme de son métabolite actif l'enaprilate, le captopril, le lisinopril, le delapril, le fosinopril, le quinapril, le spirapril, l'imidapril, le trandolapril éventuellement sous forme de son métabolite actif le trandolaprilate, le benazepril, le cilazapril, le temocapril, l'alacepril, le ceronapril, le moveltipril ou le moexipril, ainsi que leurs sels d'addition à un acide ou à une base pharmaceutiquement acceptable.

5. Association selon l'une quelconque des revendications 1 à 4 **caractérisée en ce que** l'inhibiteur de l'enzyme de conversion de l'angiotensine est le périndopril de formule (B) ainsi que ses sels d'addition à un acide ou à une base pharmaceutiquement acceptable.

6. Association selon la revendication 5 **caractérisée en ce que** le périndopril de formule (B) est sous la forme d'un sel de tertbutylamine ou d'arginine.

7. Composition pharmaceutique contenant comme principe actif une association selon l'une quelconque des revendications 1 à 6 en combinaison avec un ou plusieurs excipients ou véhicules inertes pharmaceutiquement acceptables.

8. Composition pharmaceutique selon la revendication 7 **caractérisée en ce que** la masse totale des principes actifs est répartie entre 60 et 90 % pour le composé (A) et entre 10 et 40 % pour le périndopril de formule (B).

9. Composition pharmaceutique selon l'une quelconque des revendications 7 ou 8 utile pour le traitement des complications vasculaires liées au diabète, aux maladies athéro-thrombotiques, à l'hyperlipidémie, à l'hypertension, aux maladies veineuses chroniques, à l'inflammation, au syndrome métabolique lié à l'obésité, ou au cancer.

10. Composition pharmaceutique selon la revendication 9 utile pour le traitement des complications cardiovasculaires et cérébrovasculaires liées au diabète, aux maladies athéro-thrombotiques, à l'hyperlipidémie, à l'hypertension, aux maladies veineuses chroniques , à l'inflammation, au syndrome métabolique lié à l'obésité, ou au cancer

11. Composition pharmaceutique selon l'une quelconque des revendications 9 ou 10 utile pour le traitement de l'infarctus du myocarde, les accidents vasculaires cérébraux, les anévrismes aortiques ou l'artérite des membres inférieurs.

12. Composition pharmaceutique selon la revendication 9 utile pour le traitement de la néphropathie liée au diabète, à l'hypertension ou aux maladies inflammatoires.

13. Composition pharmaceutique selon la revendication 9 utile pour le traitement de la néphropathie ou de la rétinopathie diabétique.

14. Composition pharmaceutique selon l'une quelconque des revendications 7 ou 8 utile pour le traitement des démences.

15. Composition pharmaceutique selon la revendication 14 utile pour le traitement de la maladie d'Alzheimer ou des démences vasculaires.

16. Utilisation d'une association selon l'une quelconque des revendications 1 à 6 pour la fabrication d'un médicament destiné au traitement des complications vasculaires liées au diabète, aux maladies athéro-thrombotiques, à l'hyperlipidémie, à l'hypertension, aux maladies veineuses chroniques, à l'inflammation, au syndrome métabolique lié à l'obésité, ou au cancer.

17. Utilisation d'une association selon la revendication 16 pour la fabrication d'un médicament destiné au traitement des complications cardiovasculaires et cérébrovasculaires liées au diabète, aux maladies athéro-thrombotiques, à l'hyperlipidémie, à l'hypertension, aux maladies veineuses chroniques, à l'inflammation, au syndrome métabolique lié à l'obésité, ou au cancer.

18. Utilisation d'une association selon l'une quelconque des revendications 16 ou 17 **caractérisée en ce que** les maladies athéro-thrombotiques sont l'infarctus du myocarde, les accidents vasculaires cérébraux, les anévrismes aortiques ou l'artérite des membres inférieurs.

19. Utilisation d'une association selon la revendication 16 **caractérisée en ce que** les complications vasculaires sont la néphropathie liée au diabète, à l'hypertension ou aux maladies inflammatoires.

20. Utilisation d'une association selon la revendication 16 **caractérisée en ce que** les complications vasculaires sont la néphropathie ou la rétinopathie diabétique.

21. Utilisation d'une association selon l'une quelconque des revendications 1 à 6 pour la fabrication d'un médicament destiné au traitement des complications vasculaires liées au diabète.

22. Utilisation d'une association selon l'une quelconque des revendications 1 à 6 pour la fabrication d'un médicament destiné au traitement des démences.

23. Utilisation d'une association selon la revendication 22 **caractérisée en ce que** les démences sont la maladie d'Alzheimer ou les démences vasculaires.
